# EUROPEAN PATENT APPLICATION

(11) **EP 3 290 075 A1**
(43) Date of publication of application: **07.03.2018**
(21) Application number: 17188463.8
(22) Date of filing: 30.08.2017
(51) Int. Cl.: A61M 5/315, A61M 5/142, A61M 5/20

(54) **INJECTOR WITH VARIABLE DOSAGE**

(30) Priority: 01.09.2016 US 201615254638
(71) Applicant: Medimop Medical Projects Ltd., 43665 Ra'anana (IL)
(72) Inventor: Filman, Reuven Y., 42560 Netanya (IL); Bar-El, Yossi, 71947 Beit Arye (IL)
(74) Representative: Petraz, Gilberto Luigi

(57) **Abstract**

Drug delivery devices of a standard size are adapted for injecting different doses. The device comprises an injection needle, a reservoir having an available volume, and a plunger for evacuating the volume into the needle for injection. A spacer is placed with the plunger in the reservoir to take up some of the available volume and leave a residual amount of available volume in the reservoir to correspond to a currently desired dose size. The spacer may be selected from different sizes corresponding to different doses.

## Description

### BACKGROUND OF THE INVENTION

The present invention, in some embodiments thereof, relates to an injector or drug delivery device that can be used with different dosage sizes.

A drug delivery device with a bent tip syringe is a device that contains a substance to be injected and which is placed flat against the skin. A needle is extended perpendicularly from the base of the device against the skin to pierce the skin and inject the drug.

U.S. Patents 6500150, 6824529 and 6843782 disclose, a drug delivery device having a base member defining a skin-contacting surface, a syringe serving as a reservoir for the drug, and means for expelling drug from the syringe. The syringe is connected to the base member such that the longitudinal axis of the syringe is substantially parallel to the skin surface. A delivery needle is in communication with the syringe. The needle has an angled bend which directs the tip of the needle substantially perpendicular to the skin-contacting surface. In use, the tip of the needle is adapted to penetrate the skin of the subject.

U.S. Patent Publication No. 20140163526 discloses that, "an automated injection device may be loaded with a standard type syringe and/or hypodermic needle. Optionally the syringe may be supplied loaded with medicine and/or covered with a sterile needle cover. The syringe may be loaded into the injector with in a sterile state with needle cover in place. Injector may include for example a fastener (for example an adhesive base). In some embodiments, the fastener may assist a user to hold injector steady on the skin of a patient for an extended period. For example, injector may be used to give injections of volume ranging between 0.5 and 3.0 ml over a time period ranging between 30 sec to 180 sec".

U.S. Patent Publication No. 20150088071 discloses an activation mechanism and a safety latch. The activation mechanism is operative to deploy a needle to protrude out of a housing, the needle having a longitudinal axis. The safety latch is movably mounted on the housing and formed with a needle opening to allow the needle to pass through. The safety latch has a first position wherein the needle is aligned to pass through the needle opening and a second position wherein the safety latch is moved with respect to the housing such that the needle is blocked from movement in a direction parallel to the longitudinal axis thereof by a portion of the safety latch distanced from the needle opening.

International Published Patent Application 2015048791 discloses a method of preparing a compound device for use. The device may include a sealed component and an active outer surface. The outer surface may be protected by a surface cover. Preparing the device may include activating the active outer surface by removing the surface cover and exposing an internal portion of the sealed component to the exterior of the device by unsealing the sealed component and synchronizing the activating and said unsealing using a coupler attached to the surface cover and the sealed component.

International Patent Publication No. 2013/115843 discloses an apparatus for autonomous variable rate delivery of a substance. In some embodiments, the delivery apparatus may be programmable. In some embodiments, the delivery apparatus may be disposable. Optionally the rate of delivery may be dependent on a temperature of a component of the apparatus. Optionally, there may be a time delay between activation of the apparatus and delivery of the substance.

Additional background art includes U.S. Patent no. 6189292. U.S. Patent Publication No. 20130253434, U.S. Patent Publication No. 2009/093,792, U.S. Patent No. 7967795, and US Patent Publication No. 2014/0188073.

The skin contacting surface is intended to make the devices easy for self-administration of the drug. The surface is placed against the skin and a button is pressed or lever released to release a needle to be inserted under the skin.

The drug injection devices are mass produced using molds and a production line and thus it is difficult to manufacture different sizes for different dosages. Nevertheless, different injections require different dosages, depending not only on the drug being used but also the height and weight of the patient.

One solution is to provide different injector/reservoirs products for each dose. Such a solution is a major manufacturing expense and is inefficient.

An alternative solution is to provide a standard size injector and for lower doses leave the reservoir partially empty.

A further solution is to fill the injector to the end but stop the injection process before emptying the reservoir. Such a solution has the disadvantage of wasting medicine that is possible very costly and also leaves the issue of safe disposal of the waste medicine and possible mishandling.

### BRIEF SUMMARY OF THE INVENTION

The present embodiments may provide a variable filler or spacer that can be inserted on the plunger in the syringe or reservoir. The plunger and all other components of the injector are standard components but for all doses less than the maximum a suitable sized filler is inserted with the plunger to take up the unneeded space in the reservoir. Then the reservoir is filled in the normal way and emptied in the normal way and the same dispenser can be used for a whole range of dosages.

According to an aspect of some embodiments of the present invention there is provided a drug delivery device for injecting a dose of a drug, said dose being up to a maximum dose size, the device comprising:
an injection needle or fluid path;
a reservoir having an available volume, the available volume defining said maximum dose size;
a plunger for evacuating said drug from said reservoir into said needle for injection; and
a spacer located with said plunger in said reservoir to take up some of said available volume and leave a residual volume in said reservoir, said residual volume defining a desired dose size smaller than said maximum dose size.

An embodiment may be provided with a plurality of different sized spacers for different set doses.

In an embodiment, said spacer is located in contact with said plunger.

The spacer may be located on a shaft of said plunger.

In an embodiment, said spacer is located with said plunger to limit a stroke of said plunger within said reservoir.

An embodiment may comprise a longitudinal surface for placing against the skin of an injectee and wherein said needle is placed to extend through said surface to inject under the skin.

An embodiment may comprise an electric actuator mechanism for pushing said plunger into said reservoir.

According to a second aspect of the present invention, there is provided a method of manufacturing drug delivery devices for a plurality of different dose sizes, the method comprising:
providing a plurality of drug delivery devices of a single predetermined device size, each drug delivery device comprising an injection needle, a reservoir having an available volume, and a plunger for evacuating a drug from said reservoir into said needle for injection;
providing spacers of a plurality of sizes, one size for each of said plurality of different dose sizes, said spacers being sized to take up some of said available volume to leave a residual volume in said reservoir;
selecting one of said plurality of different dose sizes;
selecting a spacer sized to leave a residual volume equal to said selected dose size;
fixing said selected spacer corresponding to said selected dose size inside one of said drug delivery devices thereby to adapt said selected one of said devices for said respective selected dose size.

The method may comprise:
locating said selected spacer in contact with said plunger in said reservoir; and
subsequently filling said residual volume with a drug to be injected.

The method may comprise placing said spacer on a shaft of said plunger.

The method may comprise providing said drug delivery devices with a longitudinal surface for placing against the skin of an injectee and placing said needle to extend through said surface to inject under the skin.

The method may comprise providing an electric actuator mechanism for pushing said plunger into said reservoir.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a simplified flow chart showing the use of a drug delivery device according to the present embodiments;
FIG. 2 is a simplified diagram showing a drug delivery device according to the present embodiments;
FIG. 3A is a simplified diagram showing a plunger for the device of FIG. 2 with a spacer to adapt the device for a given dose size;
FIG. 3B is a simplified diagram showing the spacer of FIG. 3A;
FIG. 4 is a simplified flow chart showing a method of manufacture of drug delivery devices of a standard size and subsequently adapted for different doses, according to embodiments of the present invention;
FIG. 5 is a simplified diagram showing an alternative drug delivery device according to the present embodiments;
FIG. 6A is a detail side view of an exemplary embodiment of the drug delivery apparatus of FIG. 5 in an open state showing a movable element of a cartridge and a coupler;
FIG. 6B is a detail rear view of an exemplary embodiment of the drug delivery apparatus of FIG. 5 in an open state showing a movable element of a cartridge and a motive element of the drug delivery apparatus;
FIG. 6C is a detail side cutaway view of an exemplary embodiment of the drug delivery apparatus of FIG. 5 in a closed state showing a movable element of a cartridge and a coupler; and
FIG. 6D is a detail rear cutaway view of an exemplary embodiment of the drug delivery apparatus of FIG. 5 in an closed state showing a coupler coupling a movable element of a cartridge to a motive element of the drug delivery apparatus.

### DETAILED DESCRIPTION OF THE INVENTION

The present invention, in some embodiments thereof, relates to an injector or drug delivery device for different dosages and more particularly but not exclusively to a dosage varying mechanism that allows a standard size device to be manufactured and used for different dosages.

Drug delivery devices of a standard size are adapted for injecting different doses. The device comprises an injection needle, a reservoir having a predetermined volume, and a plunger for evacuating the volume into the needle for injection. A spacer is placed with the plunger in the reservoir to take up some of the predetermined volume and leave a residual amount of available volume in the reservoir for a set dose size. The spacer may be selected from different sizes corresponding to different doses.

The drug delivery device as manufactured is now described. The device may be provided with a needle protruding from the syringe at an angle to the axis of the barrel of the syringe. Optionally the syringe may include a hub with a mount for a needle cap and/or a needle fitting and/or a luer fitting; for example the hub may be at an angle to the axis of the barrel of the syringe. Optionally the syringe or reservoir is configured to be pre-filled with a drug for injecting via the needle. Optionally the hub includes a built in needle and/or a needle mount and/or a mount for a sterile needle cap at an angle to the axis of the barrel of the syringe.

In some embodiments, the user removes a sterile needle cap from the prefilled sterile syringe before use. Optionally removing the needle cap activates the device and/or peels an adhesive cover and/or triggers deployment of a needle shield latch from an open position to a shielding position. In some embodiments, placing a base of the device on the skin of a subject moves the needle shield latch into a primed position. Optionally, the device discharges a drug into the subject with the axis of the prefilled syringe substantially parallel to the base of the device and/or the skin of the subject. Optionally a needle mounted on the cartridge may be inserted into the skin of the subject and/or may serve as a fluid path directly from the cartridge to the subject. Optionally, removing the device from the skin of the subject redeploys the needle shield latch and/or deactivates the device.

In some embodiments the drug delivery device may include a prefilled syringe and/or reservoir with a needle and/or hub at an angle to the axis of the barrel of the reservoir for example as described in various embodiments herein. For example, the drug delivery device may include a needle shield latch and/or a needle retraction mechanism for example as described in various embodiments herein. For example, the delivery device may include a needle cap and/or cover remover for example as described in various embodiments herein. For example, the delivery device may include a dual movement pivot for example to insert a needle into the subject as described in various embodiments herein.

In some embodiments it is intended that the needle is both inserted and removed in a way that is simple for the user. The insertion is linear and so is the removal and one way to achieve both insertion and removal is to have two springs or like sources of tension working against each other, or to insert the needle with the press of a button against the tension of a spring intended to facilitate withdrawal of the needle.

In order to provide different doses the reservoir could be made smaller, but that would require separate manufacturing runs which is inefficient, so rather than making the container smaller it is possible to lengthen the plunger rod without changing the injector. Particularly the plunger mount can be conveniently treated as a modular component and be replaced with a spacer plunger mount to reach the desired reservoir volume.

The plunger mount (or another part of the plunger rod) may thus be replaced by a modular piston rod extender that reduces the piston stroke by a known volume without changing any other parts of the larger payload injector.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

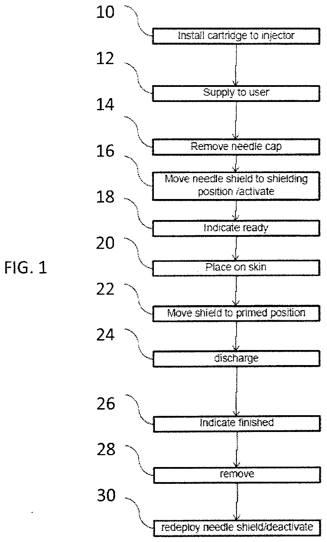

Referring now to the drawings, FIG. 1 is a flow chart illustration of a method of delivering a drug and/or shielding a needle in accordance with an embodiment of the current invention. Optionally a single use drug delivery device (for example an injector for example an auto-injector for example a patch injector, a bolus injector and/or a body worn injector) may come preloaded with a drug. For example the device may include cartridge. Optionally the cartridge includes a sterile syringe with a hub and/or sterile needle mounted at an angle to the axis of the syringe barrel. The syringe is optionally prefilled with a drug. Optionally the device may include a needle shield latch and/or a status indicator. Optionally the device may include an attachment mechanism, for example an adhesive for attaching to a subject.

In some embodiments a cartridge may be installed **10** to the delivery device before assembly and/or before being shipped to a retailer and/or a health provider and/or a user. Alternatively or additionally the cartridge may be installed **10** into the drug delivery device by a user, for example a health provider (for example a nurse and/or a pharmacist and/or a doctor and/or a health aid) and/or a subject of the injection (e.g. a patient receiving the drug) and/or a caretaker.

In some embodiments an assembled injector (with the cartridge installed **10**) may be supplied **12** to a user. Optionally as supplied **12** to the user, the cartridge and/or hub and/or the needle may be sterile and/or covered with a sterile needle cap. Optionally, the auto-injector may have a needle shield latch. For example, while the needle cap is in place the needle shield latch may be in an open position, allowing the access to the needle cap. For example, when the needle shield latch is in the open position there may be space for the cap and/or a cap remover to protrude out of the auto-injector. For example, in the open position, the needle shield latch may retract. In some embodiments the needle shield latch may pivot and/or slide from one position and/or state to another. Alternatively or additionally the cartridge and/or the injector may be supplied **12** to the user separately and/or may be assembled by the user.

In some embodiments, before use of an injector, the needle cap may be removed **14.** For example, the needle cap may be pulled off of the cartridge through an aperture in the injector housing. Optionally removing **14** the needle cap may cause the delivery device to be activated **16** and/or the needle shield to move to a shielding position. For example, the cap and/or a removal tool may be connected to a switch and/or a battery insulator and/or an adhesive protector. Removing the cap optionally activates the switch and/or causes removal of the battery insulator and/or removal of the adhesive protector. Once activated **16,** the delivery device optionally indicates **18** that it is ready to be placed on a subject. Optionally, once the needle shield is in the shielding position it may prevent replacement of the needle cap. Optionally, when the needle shield is in the shielding position it may prevent exposure of the needle. For example, in the shielding position, the needle shield may block the needle tip from extending out of the injector. Alternatively or additionally, when the shield is in the shielding position the needle extension mechanism may be locked and/or disabled thereby preventing extension of the needle out of the injector and/or in the shielding position the needle shield may shield an extended needle tip.

In some embodiments, the injector is placed **20** on the skin of a subject. For example, the delivery device may be placed **20** on the skin of the subject after activation **16.** Optionally placement on the skin causes the needle shield to move **22** to a primed position. For example, the shield may be pushed by the skin of the subject into the primed position. Movement **22** of the shield into the primed position may cause extension of a needle from the injection device and/or insertion of the needle through the skin of the subject. For example, the shield may collapse inward exposing the needle and/or allowing the needle to enter the subject and/or movement **22** of the shield may trigger an insertion mechanism. Alternatively or additionally, movement **22** of the shield into the primed position may open a path for the needle to exit the injector. For example, movement **22** of the shield into the primed position may align a hole in the shield to the needle allowing the needle to be extended out of the device, for example through the hole in the needle cap. Alternatively or additionally, an additional step may cause insertion of the needle, for example pushing a button and/or pushing a portion of the injector towards the skin of the subject.

After insertion of the needle, a drug may be delivered **24** into the subject. For example the needle may be hollow and the drug may be delivered **24** through the needle into the subject. Optionally, once injection has completed the device may indicate **26** that injection is finished and/or that the device may be removed. Optionally when a malfunction occurs (for example an occlusion of the fluid path) an indicator may indicate that a malfunction has occurred and/or that the injector should be removed. In some embodiment, the needle may retract upon completion of injection and/or upon certain malfunctions. Alternatively or additionally, the needle may remain extended after delivery **24.**

In some embodiments, after delivery **24** and/or error and/or when indicated to do so, a user may remove **28** the injector from the skin of the subject. In some embodiments when the injector is removed **28,** the needle shield may redeploy **30.** Redeployment **30** of the needle protector may protect the needle and/or shut down the device. Alternatively or additionally, needle retraction may protect the needle. Alternatively or additionally, the device may shut down and/or be disabled immediately after completion of delivery **24** and/or upon certain malfunctions. Alternatively or additionally, the device may shut down and/or be disabled after a certain time period after the completion of delivery **24** and/or upon certain malfunctions.

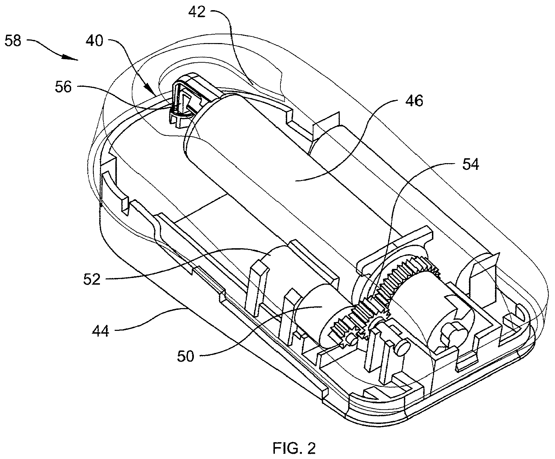

Reference is now made to FIG. 2, which is a simplified diagram illustrating a drug delivery device **40.** The drug delivery device includes upper and lower longitudinal surfaces **42** and **44,** wherein lower longitudinal surface **44** is intended to be placed against the skin. Reservoir **46** contains a drug to be injected and a plunger, not visible within the reservoir, empties the drug into the needle for injection. The plunger is operated by a plunger operation mechanism including a small electric motor **50** and battery **52,** and a motion translation arrangement **54** consisting of interlocking cog wheels. A needle insertion mechanism **56** is located at a forward end **58** of the drug delivery device **40** to insert a needle (not visible in this view) into the skin of a patient and withdraw the needle afterwards. The mechanism pushes a plunger rod which in turn pushes the plunger.

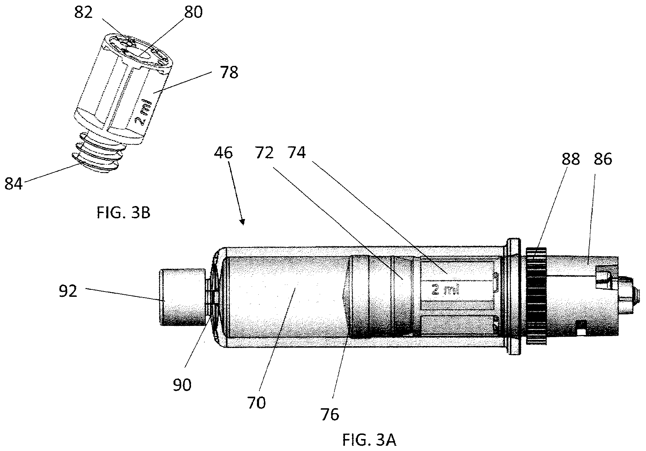

Reference is now made to FIG. 3A, which is a simplified diagram showing the reservoir 46 of the present embodiments. The reservoir has an internal space 70 which is of a fixed size. Plunger 72 extends into the space and spacer 74 is fitted behind the plunger to vary the starting position of the plunger head 76 and thus varying the available space in the reservoir. In the example shown in the figure, the spacer is of size 2ml, and thus can be used to adjust say a 15ml reservoir for a 13ml dose.

The reservoir is manufactured in a standard manufacturing process which only makes one size of reservoir. The spacers are simple moldings and thus can be made of different sizes. Thus any dose size up to the total size of the reservoir can be accommodated by selecting a suitable size of spacer.

Referring now to FIG. 3B and reference numeral **78** denotes the spacer detached from the plunger. The spacer has a hole **80** to fit over the shaft of plunger **72.** Particularly the plunger mount can be conveniently treated as a modular component and be replaced with the alternative spacer plunger mount to reduce the reservoir volume by the desired amount to meet the dosage requirement.

The plunger mount (or another part of the plunger rod) may thus be replaced by a modular piston rod extender that reduces the piston stroke by a known volume without changing any other parts of the larger payload injector.

Lugs **82** lock the orientation of the spacer when fitted and screw base **84** allows the spacer to be screw-fitted onto the plunger.

The plunger base 86 includes a cog 88 for engaging the motor to operate the plunger and at the head end, neck **90** holds a neck cover **92** which provides a sterile connection to an injection needle.

The drug delivery device is thus manufactured in a standard size along with spacers of different sizes. For any given dose a drug delivery device is then matched with the appropriate spacer to provide the exact dose and no drug is wasted.

The drug delivery device injects a dose of a drug. As discussed, the device comprises an injection needle, and a reservoir **46** having an available volume that is preset in a manufacturing process. The available volume is the volume of the reservoir minus the space taken up by the plunger head itself. The manufacturing is typically large scale. Plunger **72** evacuates the content of the reservoir into the needle for injection. The spacer **74** is fixed to the plunger to fill unwanted reservoir volume and thus adapt the available volume of the reservoir to leave a residual volume for the required dose. The spacer allows the available volume to be reduced even though the reservoir itself remains the same size so that the same reservoir can be used for a range of doses. Different spacers of different sizes are prepared and made available for selection so that the reservoir can provide any desired dose up to a maximum size.

As shown in FIG. 3A, the spacer **74** is located in contact with the plunger **72.** As shown the spacer is fitted over the shaft of the plunger to displace the plunger head from the end of the reservoir, and the spacer is typically attached to telescoping screw assembly on one side and may be connected to the plunger on the other side in order to progress linearly. However the spacer has already played its part in defining the dose size.

As an alternative the spacer **74** could be located on the head **76** of the plunger. In such a case the spacer is pushed by the plunger into the reservoir and acts as an extended head of the plunger.

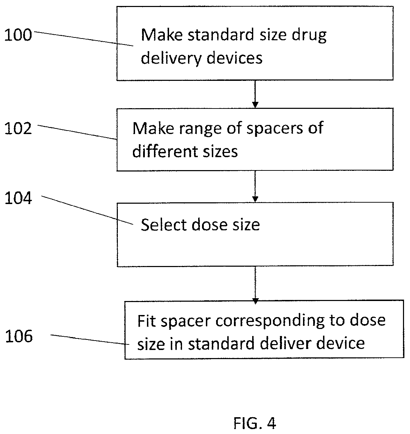

Reference is now made to Fig. 4, which is a simplified diagram showing a method of manufacturing drug delivery devices for a plurality of different dose sizes.

In box **100** a standard manufacturing process, typically involving molds for parts and a production line, produces drug delivery devices of a single preset device size. As above, each drug delivery device comprises an injection needle, a reservoir having a predetermined available volume, and a plunger for evacuating a drug from the available volume into the needle for injection.

In box **102,** spacers of a range of sizes are made, there being one size for each different dose size that is likely to be wanted. Each spacer is sized to take up some of the predetermined available volume to leave a residual volume in the reservoir corresponding to one of the dose sizes.

At some point a particular dose size is selected - box **104.** In box **106** the corresponding spacer is selected and inserted into one of the devices. Thus the device is adapted for the desired dose size.

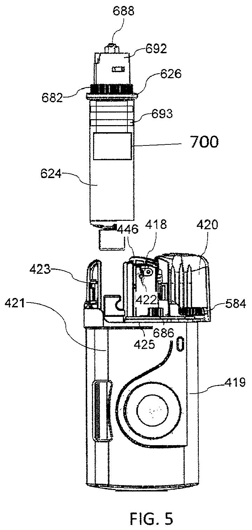

In Fig. 5, an exemplary embodiment of an optional cartridge **624** is illustrated. In Fig. 5, cartridge **624** is shown ready for insertion into channel **421.**

In some embodiments, cartridge **624** is in the form of a syringe barrel. Optionally, cartridge **624** includes a flange **626.** Flange **626** may be, for example, similar to flanges on standard syringes. For example, flange **626** may be at or towards the rear end (opposite the tip) of the syringe.

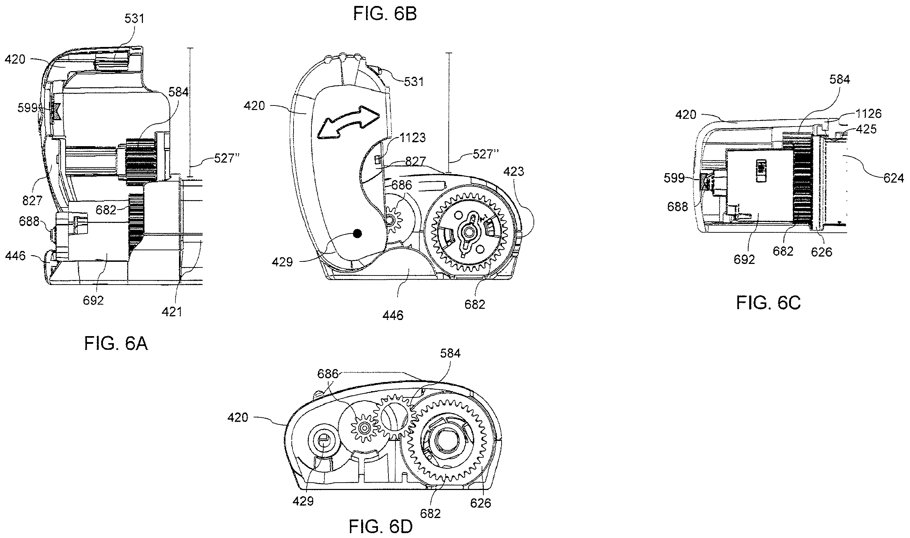

In some embodiments, an optional telescoping assembly **692** may be supplied. Assembly **692** may optionally be fitted to the open end of cartridge **624.** Telescoping assembly **692** may optionally be driven by a movable element **682** which may, for example, include a gear. Optionally, the gear revolves telescoping assembly **692.** Optionally when door **420** is closed, coupler **584** connects movable element **682** to a motive element **686** (for example see Fig. 6D). Motive element, **686** may optionally supply torque to drive movable element **682.** Assembly **692** may include a projection **688.** For example, projection **688** may be configured to fit in a notch in door **420** (for example hub **599** of Fig. 6C). Optionally, projection **688** may fit into hub **599** when door **420** is closed. When projection **688** is fit into hub **599,** projection **688** may optionally support the rear end of assembly **692.** When movable element **682** revolves, projection **688** may optionally revolve in the hub **599.** Telescoping assembly **692** may optionally drive a plunger **700.** Plunger **700** may, for example, be driven by telescoping assembly **692** into cartridge **624** to deliver a drug.

As shown in Fig. 5, the spacer **693** is located in contact with the plunger **700.** As shown the spacer is fitted between the shaft of the Telescoping assembly 692 and plunger 700 to displace the plunger towards the end of the reservoir, the spacer is typically attached to telescoping screw assembly on one side and to the plunger on the other side in order to progress linearly. However the spacer has already played its part in defining the dose size.

As an alternative the spacer **693** may be fixed on the head of the plunger **700.** In such a case the spacer is pushed together with plunger into the reservoir and acts as an extended head of the plunger **700.**

Figs. 6A-D illustrate various views of the exemplary transmission mechanism for a drug delivery apparatus of Fig. 5. In the exemplary embodiment, a coupler **584** mounted on door **420** transmits power from a motive element **686** of the apparatus to a movable element **682** of cartridge **624.**

Reference is now made to Fig. 6A, which is a side view of an exemplary apparatus according to claim with door **420** in the open state. In the exemplary embodiment coupler **584** includes a gear. In the exemplary apparatus, movable element **682** includes a gear. It can be seen that, with door **420** in the open state coupler **584** is optionally disengaged from movable element **682.**

Reference is now made to Fig. 6B, which is a rear view of exemplary apparatus with door **420** in the open state. In the exemplary apparatus, motive element 686 includes a gear. When door **420** is in the open state, motive element **686** and movable element **682** are optionally disengaged. In Fig. 6B a latch **1123** can be seen. Optionally latch **1123** latches door **420** to rear support **446** when door **420** is in a closed state.

Reference is now made to Fig. 6C, which illustrates a side cutaway view of exemplary drug delivery apparatus in a closed state. Fig. 6C illustrates an optional hub **599.** In some embodiments, hub **599** may be mounted on an inside surface of door **420.** When door **420** is in a closed state, hub **599** may optionally support projection **688.** Projection **688** may optionally protrude from cartridge **624.** For example, projection **688** may protrude from a rear end of telescoping assembly **692.** When door **420** is in a closed state, hub **599** may optionally support the axial force when the telescoping assembly expands, for example forcing a plunger **700** into cartridge **624.** Optionally, forcing plunger **700** into cartridge **624** may cause delivery of a drug.

In some embodiments, when door **420** is in a closed state a supporting structure may engage and/or support door **420.** For example, in Fig. 6C a lip **1126** on door **420** is shown engaged to a groove **425** on apparatus housing **419.** When engaged to lip **1126,** groove **425** may support door **420.**

Reference is now made to Fig. 6D, which illustrates a rear cutaway view of an exemplary apparatus with door **420** in the closed and/or permanently locked state. Optionally when door **420** is in the closed and/or locked state coupler **584** engages motive element **686** with movable element **682.** Optionally, movable element **682** may transmit power to telescoping assembly **692.** Optionally, power transmitted from motive element **686** through coupler **584** to movable element **682** may cause telescoping assembly **692** to expand, pushing plunger **700** into cartridge **624** to the extent permitted by spacer **693** (Fig. 5), thus delivering the intended dose level of the drug.
It is expected that during the life of a patent maturing from this application many relevant drug delivery devices and injection mechanisms will be developed and the scopes of the corresponding terms are intended to include all such new technologies *a priori.*

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting. It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the appended claims.

## Claims

1. A drug delivery device for injecting a dose of a drug, said dose being up to a maximum dose size, the device comprising:
an injection needle or fluid path;
a reservoir having an available volume, the available volume defining said maximum dose size;
a plunger for evacuating said drug from said reservoir into said needle for injection; and
a spacer located with said plunger in said reservoir to take up some of said available volume and leave a residual volume in said reservoir, said residual volume defining a desired dose size smaller than said maximum dose size.

2. The drug delivery device of claim 1, provided with a plurality of different sized spacers for different set doses.

3. The drug delivery device of claim 1, wherein said spacer is located in contact with said plunger.

4. The drug delivery device of claim 1, wherein said spacer is located on a shaft of said plunger.

5. The drug delivery device of claim 1, wherein said spacer is located with said plunger to limit a stroke of said plunger within said reservoir.

6. The drug delivery device of claim 1, comprising a longitudinal surface for placing against the skin of an injectee and wherein said needle is placed to extend through said surface to inject under the skin.

7. The drug delivery device of claim 6, comprising an electric actuator mechanism for pushing said plunger into said reservoir.

8. A method of manufacturing drug delivery devices for a plurality of different dose sizes, the method comprising:
providing a plurality of drug delivery devices of a single predetermined device size, each drug delivery device comprising an injection needle, a reservoir having an available volume, and a plunger for evacuating a drug from said reservoir into said needle for injection;
providing spacers of a plurality of sizes, one size for each of said plurality of different dose sizes, said spacers being sized to take up some of said available volume to leave a residual volume in said reservoir;
selecting one of said plurality of different dose sizes;
selecting a spacer sized to leave a residual volume equal to said selected dose size;
fixing said selected spacer corresponding to said selected dose size inside one of said drug delivery devices thereby to adapt said selected one of said devices for said respective selected dose size.

9. The method of manufacture of claim 8, comprising:
locating said selected spacer in contact with said plunger in said reservoir; and
subsequently filling said residual volume with a drug to be injected.

10. The method of manufacture of claim 9, comprising placing said spacer on a shaft of said plunger.

11. The method of manufacture of claim 8 comprising providing said drug delivery devices with a longitudinal surface for placing against the skin of an injectee and placing said needle to extend through said surface to inject under the skin.

12. The method of manufacture of claim 8, comprising providing an electric actuator mechanism for pushing said plunger into said reservoir.
